# EUROPEAN PATENT APPLICATION

(11) **EP 2 540 228 A1**
(43) Date of publication of application: **02.01.2013**
(21) Application number: 11747497.3
(22) Date of filing: 25.02.2011
(51) Int. Cl.: A61B 17/06, A61B 17/3211

(54) **SURGICAL KNIFE AND MANUFACTURING METHOD THEREOF**

(30) Priority: 25.02.2010 JP 2010040003
(71) Applicant: Mani, Inc., Tochigi 321-3231 (JP)
(72) Inventor: MATSUTANI, Kanji, Utsunomiya-shi Tochigi 321-3231 (JP)
(74) Representative: Barth, Stephan Manuel
(86) International application number: PCT/JP2011/054258
(87) International publication number: WO 2011/105541

(57) **Abstract**

A surgical cutlery and a manufacturing method thereof, which prevent over-etching and reduce pierce resistance, are provided. The manufacturing method of the surgical cutlery made of a round bar material 15 of austenitic stainless steel with a fibrous structure includes the steps of: forming a working portion 16 of the cutlery from the round bar material 15 for piercing or cutting a living tissue, wherein the fibrous structure runs along an axis a; forming a wheel mark 24 on the working portion of the surgical cutlery; and etching the working portion 23 after etching. The etching is performed to the limit that allows creation of a plurality of craters on the working portion, but does not allow creation of grooves along the fibrous structure. Afterwards, application of silicone is performed on the resulting surface.

## Description

### [Technical Field]

The present invention relates to a surgical cutlery, such as a surgical suture needle or a surgical knife, and a manufacturing method thereof.

### [Background Art]

Typical surgical suture needles include round needles having a round cross-sectional form and a sharp front end, and cutting needles having a polygonal cross-sectional form, such as a triangular form, and a pointy, sharp front end. The round needles and the cutting needles use round material made of austenitic stainless steel as a raw material. The austenitic stainless steel does not rust and is thereby suitable for surgical equipment; however, on the other hand, it is soft and cannot be quenched.

Therefore, an austenitic stainless steel wire rod is stretched and work hardened, so as to attain a predetermined hardness. The stainless steel wire that is stretched and work hardened as such has a crystalline structure spreading in strips along the length of the wire. This is referred to as a fibrous structure.

A suture needle is manufacture from such a stainless steel wire in the following manner. First, the stainless steel wire that has a fibrous structure is cut to a predetermined length, and a blind hole is formed on an end along the axis of the stainless steel wire. Alternatively, a spring eye may be formed. The other end is ground and sharpened. At this time, in the case of the round needle, the raw material is rotated and ground so as to make a round cross-section, and in the case of the cutting needle, each side is ground separately to make a pyramid. Curvature is then given, thereby forming the suture needle shape.

The round needles do not damage surrounding living tissue, and are thus mainly used for suturing internal organs and the like, and the cutting needles have an edge functioning as a cutting blade and great incision power, and are thus used for suturing outer skin.

On the other hand, the surgical knife results from cutting the same stainless steel wire with a fibrous structure to a predetermined length, pressing an end flat, and molding the pressed portion into a knife shape using a different press machine, and forming a cutting blade through grinding.

The suture needle and the surgical knife have problems with pierce resistance and sharpness, and therefore various measures have been taken to reduce pierce resistance and improve sharpness.

Application of silicone is often performed as one of these measures. This is because a silicone film reduces frictional resistance.

In addition, a suture needle having an eye running almost orthogonal to the central axis thereof and eye running along the length of the central axis is proposed in Patent Document 1 (JP 3140508). This is attained by polishing the raw material having a crystalline fibrous structure in a direction orthogonal to the length of the fibrous structure, making grooves running orthogonal to the length of the fibrous structure by abrasive grains, and then carrying out processing such as electrolytic polishing and chemical polishing, thereby etching the boundary of the fibrous structure so as to make fibrous grooves. The grooves running along the central axis result from performing electrolytic polishing on the pattern of the fibrous structure, the grooves running orthogonal to the central axis are made by abrasive grains during polishing. Silicone is applied to the suture needle that has vertical and horizontal grooves and the suture needle is used.

### [Prior Art Documents]

### [Patent Documents]

Patent Document 1 : JP 3140508

### [Disclosure of Invention]

### [Problem to be Solved by the Invention]

However, the method disclosed in the aforementioned Patent Document 1 has a problem of the possibility of excessive etching, the needlepoint becoming too thin and easy to bend, the edge curling up, and actually increasing pierce resistance. The surgical knife also has a problem that the cutting blade becomes too thin, the edge curls up, and the sharpness decreases.

In light of these conditions, the present invention aims to provide a surgical cutlery, such as a sharp suture needle or a surgical knife, allowing unchanging prevention of over-etching and reduction in pierce resistance, and a manufacturing method thereof.

### [Means of Solving the Problem]

In order to attain the object of the present invention, a surgical cutlery according to the present invention is characterized in that it is made of an elongated material of austenitic stainless steel with a fibrous structure running along the length of the material; wherein a wheel mark is formed on a working portion of the cutlery, which pierces or cuts a living tissue, and etching forms multiple craters on the wheel mark, with grooves running along the fibrous structure not being exposed. Furthermore, a configuration formed by applying silicone to the surface on which the craters are formed may be provided. Moreover, a configuration having the wheel mark running along an axis orthogonal to the axis of the fibrous structure may be provided. Herein, the elongated material denotes a material with a comparatively large aspect ratio, such as a round bar material, a rectangular bar material, a hoop formed by coiling a long plate material.

In order to attain the object of the present invention, a manufacturing method of a surgical cutlery made of an elongated material of austenitic stainless steel with a fibrous structure is characterized in that it includes the steps of: forming a working portion of the cutlery from the material for piercing or cutting a living tissue, wherein the fibrous structure runs along the length of the cutlery; forming a wheel mark by polishing the working portion; and etching the polished working portion; wherein the etching is performed to the limit that allows creation of a plurality of craters on the working portion, but does not allow creation of grooves along the fibrous structure. Furthermore, a configuration where the polishing is performed along an axis orthogonal to the central axis of the cutlery may be provided. Moreover, a configuration formed by applying silicone to the surface to which the craters are exposed may be provided.

### [Result of the Invention]

According to the surgical cutlery of the present invention, since the etching after a wheel mark is formed is performed to the limit that does not allow creation of grooves along the fibrous structure, the surgical cutlery, such as a suture needle and a surgical knife, having low pierce resistance but excellent sharpness, with the needle point and the cutting blade not being too thin may be obtained.

Furthermore, since concave craters are formed on the surface of the working portion, application of silicone to the working portion makes the silicone enter the craters, thereby increasing adherence of a silicone film, making it difficult to come off easily. Moreover, pierce resistance of the suture needle will decrease, and sharpness of the knife increases.

### [Brief Description of Drawings]

FIGS. 1(a) to 1(f) are diagrams describing a manufacturing method for a cutlery formed from a round bar material as a surgical knife according to the present invention;
FIGS. 2(a) to 2(e) are diagrams describing a manufacturing method for a suture needle as the surgical cutlery according to the present invention;
FIG. 3 describes etching according to the present invention, where 3(a) is a magnified view illustrating a working portion of the suture needle before etching, 3(b) is a magnified view illustrating the working portion when it has been appropriately etched, and 3(c) is a magnified view illustrating the working portion when it has been over-etched; and
FIGS. 4(a) to 4(e) are diagrams describing a manufacturing method for a slit knife formed from a hoop as the surgical cutlery according to the present invention.

### [Best Mode for Carrying Out the Invention]

An embodiment of the present invention is described while referencing the attached drawings.

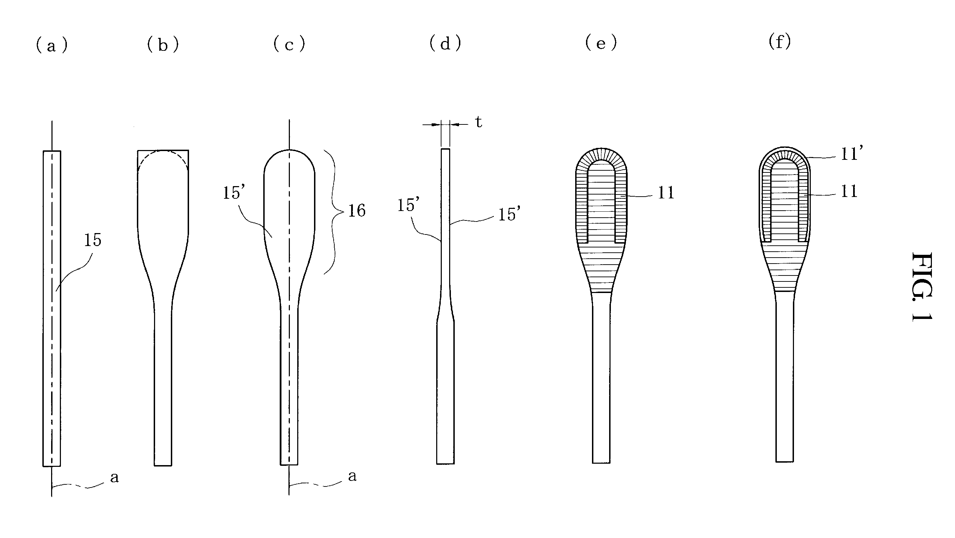

A manufacturing method for a knife as the surgical cutlery according to the present invention will be described next with reference to FIG. 1. To begin with, a round bar material 15 as the elongated material shown in FIG. 1(a) is cut at a predetermined length. This round bar material 15 is mainly made of austenitic stainless steel, such as SUS 304, 302, or 420, and the fat round bar material is wire-drawn several times, into an elongated fibrous structure along the length of the round bar material 15, namely along a central axis a.

Note that the fibrous structure is not normally visible, but may be visible by generating concavo-convex grooves along the fibrous structure through erosion when it has been etched, for example.

Next, as shown in FIG. 1(b), the front edge is pressed flat and the excessive portion as indicted by a dotted line is cut, roughly resulting in a knife form. As shown in FIGS. 1(c) and 1(d), a flattened surface 15' is polished from both sides and made into a smooth surface so as to determine thickness t. At this time, if the polishing axis crosses the central axis a, it is easy to determine whether grooves have generated along the fibrous structure in the etching process. It is most effective if the polishing axis crosses the fibrous structure at 90 degrees. The periphery thereof is then polished so as to form a roughly ground surface 11, as shown in FIG. 1(e). The periphery of the roughly ground surface 11 is polished thereafter to form a finished surface 11' and form a sharp cutting blade. The shape of the knife may be precisely established through this polishing. Moreover, while the polishing axis may cross the fibrous structure in a direction orthogonal to the cutting blade, it may also be parallel to the fibrous structure at the front edge of the cutting blade, for example.

In the above structure, a flat cutting portion at the front edge where a cutting blade is formed is a portion for incising a living organism, and is called a working portion 16. Etching is performed next; however, this will be described later.

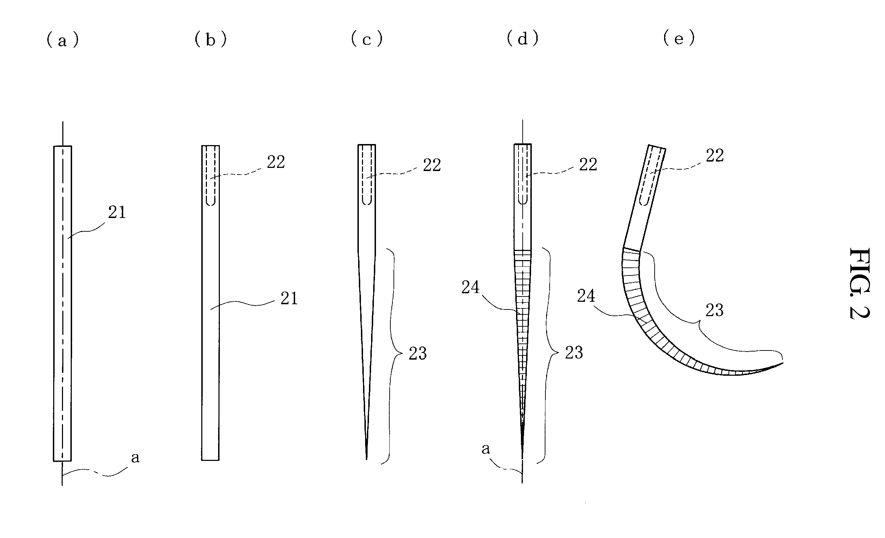

A manufacturing method for a suture needle as the surgical cutlery according to the present invention will be described next with reference to FIG. 2. To begin with, a raw, round bar material 21 shown in FIG. 2(a) is cut at a predetermined length. This raw, round bar material 21 is mainly made of austenitic stainless steel, such as SUS 304, 302, or 420, and the fat round bar material is wire-drawn several times so as to have a long and thin fibrous structure, similarly to the knife.

A blind hole 22 is then formed in the axial direction from an end as indicated by a dotted line in FIG. 2(b). Formation of this blind hole 22 is not particularly limited and may be carried out using a drill or a laser processing method which are well-known. A tapered form is then formed on the other end so as to form a working portion 23 (needle point), as shown in FIG. 2(c). In the case of a round suture needle, the round bar material 21 as the material is rotated around the central axis a and ground. In the case where the suture needle is a cutting needle such as a triangular needle, it is pressed and made into a pyramid. When using it as a suture needle, the working portion 23 pierces the living tissue.

Wheel marks 24 orthogonal to the central axis a are then formed through polishing, as shown in FIG. 2(d). This polishing may double as the grinding for forming the working portion 23 described in FIG. 2(c). The polishing axis may be the same as direction (along the central axis a) of the pattern of the fibrous structure; however, if it crosses the central axis a, it is easy to determine whether grooves of the fibrous structure have been exposed in the etching process described later. It is most effective if the polishing axis crosses the pattern of the fibrous structure at 90 degrees. The working portion is then bent so as to form a suture needle, as shown in FIG. 2(e).

A knife is formed as shown in FIG. 1(f), the suture needle is formed as shown in FIG. 2(e), and etching is then performed. While etching in this embodiment is carried out through electrolytic polishing, chemical polishing may be performed.

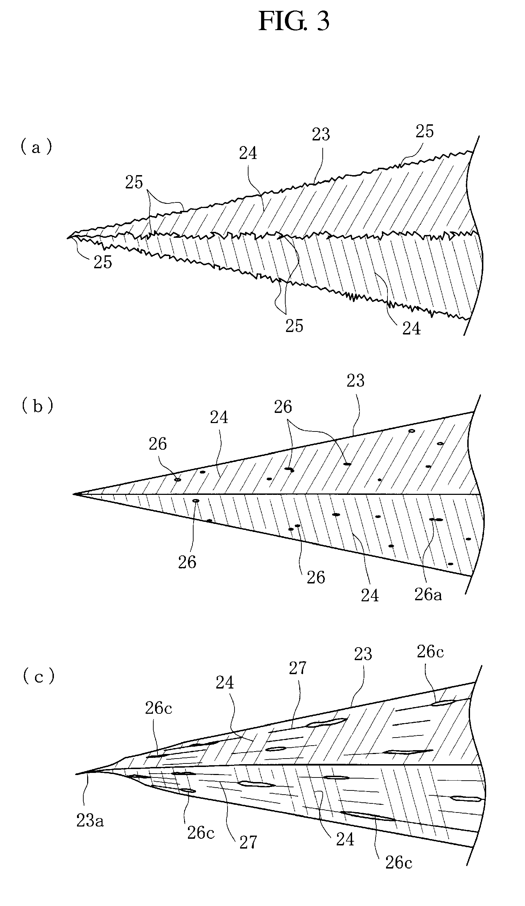

FIG. 3 describes etching using a triangular suture needle as an example, where FIG. 3(a) is a magnified view of the suture needle shown in FIG. 2(e), illustrating the working portion 23 (needle point) before etching, FIG. 3(b) is a magnified view illustrating the working portion 23 when it has been appropriately etched, and FIG. 3(c) is a magnified view illustrating the working portion when it has been over-etched.

The electrolytic polishing method soaks a surgical cutlery (a knife or a suture needle) in an electrolytic solution to make an electrode, soaks another electrode in the electrolytic solution at a distance, passes electricity between the electrodes, and causes the surface of the soaked surgical cutlery to melt. Etching removes burrs resulting from abrasive polishing and sharpens the needlepoint of a suture needle, edge of a cutting needle, knife cutting blade, and the like.

The working portion shown in FIG. 3(a) includes the wheel marks 24 in a direction orthogonal to the central axis a of the suture needle. The wheel marks 24 in this embodiment cross the central axis a along the length of the fiber structure; however, crossing angle may be within a range of zero to 90 degrees. Zero degrees means parallel with the central axis a. However, when the crossing angle is zero degrees, the wheel marks 24 resulting from etching disappear and make it difficult to confirm exposure of the grooves, and thus the crossing angle is preferably greater than zero and close to 90 degrees.

Burrs 25 generated in the grinding process carried out before are left in places such as on the needlepoint edge and edge line of the working portion 23 before etching.

FIG. 3(b) is a diagram of the case where etching has been appropriately performed. The burrs 25 have dissolved and disappeared through the electrolytic polishing. Moreover, in the case of electrolytic polishing, microscopically, generation of shallow craters 26 may be confirmed. It emanates from minute quantities of impurities, such as manganese sulfite, contained in stainless steel, gas generated during electrolysis adhered to the surface, and the like. In the case of chemical polishing, in addition to the craters 26 resulting from the same gas as in electrolytic polishing, shallow and concavo-convex craters 26 are formed on the surface of the needle due to a mixture of easily polished crystal grains and not-easily polished crystal grains in the material. Moreover, the form of the craters 26 observed by a microscope is an elongated shape running along the central axis a of the fibrous structure. In this case, the craters 26 may also be formed as successive craters 26a along the central axis a. While the successive length of the craters increases as the electrolytic polishing is powerful, approximately three successive small craters are used in this example, but nothing longer is formed; wherein crater length is approximately 5 µm or less.

FIG. 3(c) illustrates the case of over-etching. If electrolytic polishing time or electric current is increased, each crater 26 generated in the working portion 23 becomes larger as illustrated by 26c, normally growing along the length of the fibrous structure. This is a state that five or more of the craters 26 shown in FIG. 3(b) run continuously. Moreover, the wheel marks 24 are dissolved, the lower fibrous structure is etched, and grooves 27 running along the length of the fibrous structure are formed. If etching continues until this stage, etching is excessive, and in the case of a suture needle, the needlepoint 23a becomes too thin and weak, and is thereby unusable. Furthermore, even in the case of a knife, the cutting blade becomes thin, bent, and blunt, and thereby cannot cut well. In addition to the needlepoint 23a, even in the case of a cutting needle such as a triangular needle, the edge line or cutting blade becomes thin and easily bendable, thereby increasing pierce resistance.

Meanwhile, in the early stage where the craters 26 are starting to form throughout the working portion 23, the needlepoint 23a of the suture needle becomes sharp, and the knife cutting blade also becomes sharp. Moreover, since etching does not continue to the fibrous structure, and the grooves 27 are not formed, there is no adverse influence to the pierce resistance or sharpness. Furthermore, while silicone is applied to the working portion 23 after it is etched, if there are craters 26 throughout the working portion 23, silicone enters the craters 26 when applying the silicone and hardens therein, thereby increasing adherence of a silicone film, which does not come off easily. As a result, it is preferable that electrolytic polishing is stopped when the craters 26 are starting to form.

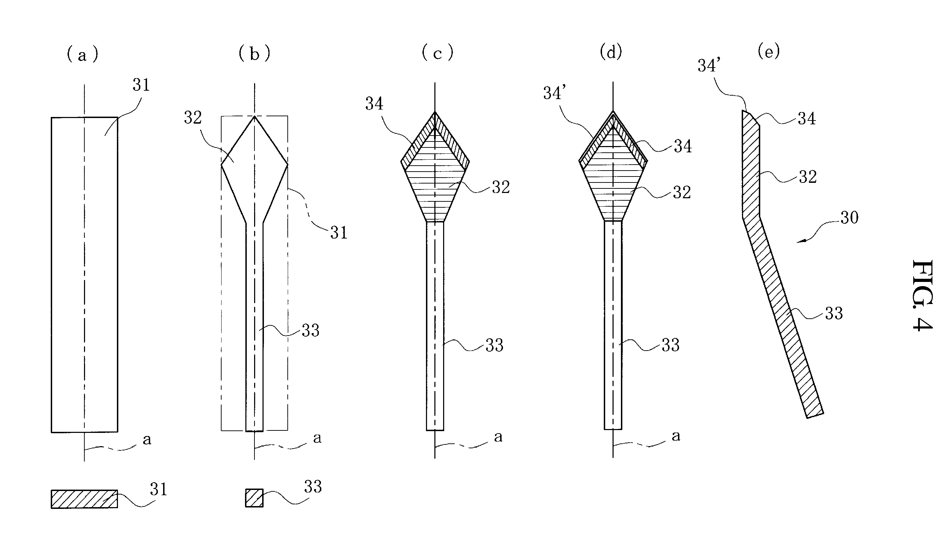

FIGS. 4(a) to 4(e) are diagrams describing a manufacturing method for a slit knife as a surgical cutlery. A slit knife 30 in this embodiment is formed from a hoop 31.

The hoop 31 has a fibrous structure and is made by stretching several times a bar material or thick plate material mainly made of austenitic stainless steel, such as SUS 304, 302, or 420, and work hardening to a predetermined hardness, and then coiling.

FIG. 4(a) illustrates a state where raw material 31 of a predetermined length is cut out from a hoop. A rectangular cross section in the bottom of the drawing indicates cross-sectional form of the hoop 31. The fibrous structure is formed along the length of the raw material 31.

The raw material 31 is cut in the form of the slit knife 30 through press working, as shown in FIG. 4(b). That is, an almost rhombus-shaped working portion 32 is formed on the front edge and a handle 33 is formed therebelow. The handle 33 is then polished on both sides to make it flat and a predetermined thickness, as shown in FIG. 4(c). At this time, if the polishing axis crosses the central axis a, it is easy to determine whether grooves have generated along the fibrous structure in the etching process. It is most effective if the polishing axis crosses the fibrous structure at 90 degrees. Moreover, the periphery thereof is then polished so as to form a rough ground surface 34, as shown in FIG. 4(c). Subsequently, a finished surface 34' is formed through polishing on the front edge of the roughly ground surface 34, as shown in FIG. 4(d). The shape of the knife may be precisely established through this polishing. Furthermore, the polishing axis crosses the cutting blade. Etching is then performed as described in FIG. 3.

Once the etching is completed, a predetermined slant is provided between the working portion 32 and the handle 33, a silicone coating is applied, and this process is completed.

Note that while in the embodiment of FIG. 4, thickness of the working portion 32 is made just a little thinner than thickness of the raw material 31 due to the polishing, the working portion 32 may be pressed and squashed to be thin. In this case, the working portion 32 undergoes work hardening and becomes harder than the raw material 31. It goes without saying that the width of the working portion 32 is wider than that of the raw material 31, and thus a narrow raw material is used. Moreover, the slit knife 30 of this embodiment may of course be formed from a round bar material as in the method of FIG. 1.

### Working Example 1

A triangular suture needle as a surgical cutlery is formed as illustrated in FIGS. 2(a) through 2(e), and an electrolytic polishing test is performed while changing the electric current. Phosphoric acid is used as an electrolytic solution.
In the following example, electrolysis time is 70 seconds and electrolysis temperature is 30 degrees Centigrade. The electrolytic solution is exchanged to a new solution every time conditions are changed.

### Case of current of 0.1 amp.

Burrs were left and craters were not confirmed. There was hardly any change before and after etching.

### Case of current of 0.2 amp.

A few burrs were gone. There was hardly any change.

### Case of current of 0.4 amp.

Many burrs were gone and a few craters were confirmed.

### Case of current of 0.6 amp.

Almost all of the burrs were gone. Several craters were formed; however, no formation of grooves running along the length of the fibrous structure was confirmed.

### Case of current of 0.8 amp.

Almost all of the burrs were gone. There were a few more craters than in the case of 0.6 amp., and successive craters along the length increased; however, no formation of grooves running along the length of the fibrous structure was confirmed.

### Case of current of 1.0 amp.

All of the burrs were gone, but several craters connected parallel to the central axis had generated, and formation of grooves along the fibrous structure had begun. Moreover, the needlepoint was narrow and unable to withstand piercing.

### Case of current of 1.2 amp.

All of the burrs were gone, but some large craters and several craters connected parallel to the central axis had generated, and grooves along the fibrous structure were noticeable. Due to the increase in electric current, etching continues to the front end, resulting in a narrow needlepoint that is unable to withstand piercing.

The above-given results say the electric current of 0.4 to 0.8 amp. is preferable, particularly favorably 0.6 amp..

Moreover, according to a test performed in conjunction with the above test, electric current is almost irrelevant to suture needle size, and when there is a difference in suture needle size, it is found that changing energization time is preferred. Furthermore, while the electrolytic solution is a phosphoric acid concentrate solution (50 to 60%), it may be mixed with sulfuric acid and made weaker.
A suture needle has been described above; however, the same holds true for another surgical cutlery such as a surgical knife or an ophthalmic knife.

### [Description of Reference Numerals]

11: roughly ground surface
15: round bar material
16: working portion
21: round bar material
22: blind hole
23: working portion
24: wheel mark
25: burr
26: crater
27: fibrous structure
a: central axis

## Claims

1. A surgical cutlery made of an elongated material of austenitic stainless steel with a fibrous structure running along the length of the material; wherein a wheel mark is formed on a working portion of the cutlery, which pierces or cuts a living tissue, and etching forms multiple craters on the wheel mark, with grooves running along the fibrous structure not being exposed.

2. The surgical cutlery of Claim 1, wherein application of silicone is performed on the state in which the craters are formed.

3. The surgical cutlery of either Claim 1 or Claim 2, wherein the wheel mark runs along an axis orthogonal to axis of the fibrous structure.

4. A manufacturing method of a surgical cutlery made of an elongated material of austenitic stainless steel with a fibrous structure, the manufacturing method comprising the steps of: forming a working portion of the cutlery from the material for piercing or cutting a living tissue, wherein the fibrous structure runs along the length of the cultlery; forming a wheel mark by polishing the working portion; and etching the polished working portion; wherein the etching is performed to the limit that allows creation of a plurality of craters on the working portion, but does not allow creation of grooves along the fibrous structure.
